# EUROPÄISCHE PATENTSCHRIFT

(11) **EP 3 390 354 B1**
(45) Veröffentlichungstag und Bekanntmachung des Hinweises auf die Patenterteilung: **29.04.2020**
(21) Anmeldenummer: 16809776.4
(22) Anmeldetag: 08.12.2016
(51) Int. Cl.: C07C 273/10, C01B 3/02, C01C 1/04

(54) **VERFAHREN ZUR BEREITSTELLUNG VON KOHLENDIOXID FÜR DIE SYNTHESE VON HARNSTOFF**
METHOD FOR THE PROVISION OF CARBON DIOXIDE FOR THE SYNTHESIS OF UREA
PROCÉDÉ PERMETTANT D'OBTENIR DU DIOXYDE DE CARBONE POUR LA SYNTHÈSE DE L'URÉE

(30) Priorität: 14.12.2015 DE 102015121756
(43) Veröffentlichungstag der Anmeldung: 24.10.2018
(73) Patentinhaber: thyssenkrupp Industrial Solutions AG, 45143 Essen (DE); thyssenkrupp AG, 45143 Essen (DE)
(72) Erfinder: MAKHYNYA, Yevgeny, 45481 Mülheim an der Ruhr (DE); JOHANNING, Joachim, 46045 Oberhausen (DE); DOSTAL, Daniela, 44225 Dortmund (DE)
(74) Vertreter: thyssenkrupp Intellectual Property GmbH
(86) Internationale Anmeldenummer: PCT/EP2016/080275
(87) Internationale Veröffentlichungsnummer: WO 2017/102546

(56) Entgegenhaltungen:
- EP-A2- 0 150 030
- WO-A1-2015/086149
- DE-A1- 3 239 605

## Beschreibung

Die Erfindung betrifft ein Verfahren für die Abtrennung von Kohlendioxid aus CO₂-haltigen Gasen und eine Vorrichtung für die Bereitstellung von Kohlendioxid zur Synthese von Harnstoff.

Die großtechnische Herstellung von Harnstoff beruht derzeit praktisch ausschließlich auf der Hochdrucksynthese aus Ammoniak und Kohlendioxid in einer Harnstoff-Anlage bei ungefähr 150 bar und ungefähr 180°C.

Beide Einsatzstoffe für die Harnstoffsynthese werden in der Regel in einer Ammoniak-Anlage bereitgestellt, welche meist in direkter Nachbarschaft zu der betreffenden Harnstoff-Anlage steht. Das für die Harnstoff-Synthese benötigte Kohlendioxid fällt dabei in der Synthesegas-Erzeugung der Ammoniak-Anlage als Bestandteil des Rohsynthesegases nach einer Reformierung an. Da das Kohlendioxid in der Ammoniak-Synthese als Katalysatorgift wirken würde, muss es aus dem Synthesegas abgetrennt werden. Im Stand der Technik ist hierfür der Einsatz regenerativer Gaswäschen bekannt, für die eine größere Zahl selektiv wirkender Lösungsmittel zur Verfügung steht.

Da das Ammoniak an der Anlagengrenze der Ammoniak-Anlage in der Regel flüssig vorliegt, kann es mit einem begrenzten energetischen und apparativen Aufwand auf das Druckniveau der Harnstoff-Anlage gebracht werden. Das Kohlendioxid fällt in der Ammoniak-Anlage jedoch gasförmig an. Es wird daher für die Druckanhebung ein ungleich größerer energetischer und apparativer Aufwand benötigt, um das Kohlendioxid auf das Druckniveau der Harnstoff-Anlage zu bringen.

Im Stand der Technik ist die Integration von Ammoniak- und Harnstoff-Anlagen bekannt, bei der die Abtrennung des Kohlendioxids mit Ammoniak bzw. Ammoniak-Wasser-Gemischen erfolgt. Das Kohlendioxid wird in der Lösung vorwiegend chemisch in Form von Karbamat- und Karbonat-Ionen gebunden und kann dann ebenfalls mit vergleichsweise geringem Aufwand durch Pumpen in die Harnstoff-Synthese eingebracht werden.

In der Vergangenheit wurden etliche Konzepte für eine prozessseitige Integration von Ammoniak- und Harnstoff-Anlagen vorgeschlagen, um sowohl den Energiebedarf für die CO₂-Verdichtung als auch den apparativen Aufwand für die Anlage insgesamt zu reduzieren. Ähnlich für alle diese Konzepte ist, dass die komplette CO₂-Menge aus dem Synthesegas mit Hilfe von prozesseigenem Ammoniak entfernt und das entstandene Gemisch ohne weiteres Aufarbeiten in die Harnstoffsynthese weitergeleitet wird. In diesem Fall spricht man über einen "vollintegrierten Ammoniak-Harnstoff-Komplex".

DE 1 668 547 offenbart ein Verfahren zur Herstellung von Ammoniak und Harnstoff, das dadurch gekennzeichnet ist, dass man Kohlendioxid, Stickstoff und Wasserstoff enthaltendes Ammoniaksynthesegas in eine erste Zone leitet, die unter solchen Bedingungen gehalten wird, dass Kohlendioxid aus dem Synthesegas in einer ammoniakhaltigen Flüssigkeit abgetrennt und ein Ammoniumkarbamat enthaltendes Kondensat erhalten wird, und dass man das restliche Ammoniaksynthesegas in eine Ammoniaksynthesezone leitet und das Kondensat in eine zweite Zone leitet, die unter zur Herstellung von Harnstoff aus dem Kondensat geeigneten Bedingungen gehalten wird. Das ist ein typisches Beispiel eines vollintegrierten Verfahrens, dabei findet keine direkte CO₂-Abtrennung statt.

DE 26 13 102 C2 offenbart ein Verfahren zur gleichzeitigen Herstellung von Ammoniak und Harnstoff, bei dem ein beim Reformieren von Kohlenwasserstoffen und anschließender CO-Umwandlung erhaltenes Gasgemisch aus Kohlendioxid, Stickstoff und Wasserstoff einer Absorption des Kohlendioxids mittels Ammoniaklösung, die bei einer Absorption des Ammoniaks aus der Ammoniaksynthese mittels Wasser anfällt, zugeführt und die so gebildete Ammoniumkarbamatlösung in die Harnstoffsynthese geführt wird.

Bei diesen beiden Verfahren werden die Ammoniumkarbamatlösungen direkt zur Harnstoffsynthese weitergeleitet. Detaillierte Untersuchungen haben aber gezeigt, dass auf diesem Weg keine energetisch günstige Gesamtlösung realisiert werden kann. Die Harnstoffsynthese aus Ammoniak und Kohlendioxid ist insgesamt exotherm. Sie besteht aus der relativ stark exothermen und verhältnismäßig schnellen Reaktion der Edukte zu Ammoniumkarbamat sowie der deutlich langsameren und endothermen Zersetzung des Karbamates zu Harnstoff und Wasser. Eine gute Energieeffizienz des Gesamtverfahrens lässt sich nur erreichen, wenn die freiwerdende Reaktionswärme der Karbamatbildungsreaktion für die Harnstoffbildung genutzt wird.

Darüber hinaus ist davon auszugehen, dass die Inbetriebnahme einer integrierten Anlage aus Ammoniak- und Harnstoff-Anlage komplex ist und ein separater Betrieb von Ammoniak- oder Harnstoff-Anlage nicht realisierbar wäre.

Bei einer CO₂-Wäsche mit Ammoniak- bzw. Ammoniak/Wasser-Gemischen wird bei der Karbamatbildung viel Wärme freigesetzt. Wasser könnte viel effektiver als Ammoniak die Wärme aufnehmen, ist aber nur wenig oder überhaupt nicht vorhanden. Für die Rückhaltung des Ammoniaks muss daher gekühlt werden. Bei den bisher vorgeschlagenen Konzepten muss diese Wärme aber größtenteils über Kühlwasser ungenutzt abgeführt werden. Die vom Harnstoff-Reaktor benötigte Wärmemenge muss dann zusätzlich bereitgestellt werden und die Energiebilanz des Prozesses wird ungünstiger. Des Weiteren wird bei den im Stand der Technik beschriebenen Verfahren eine erhebliche zusätzliche Wassermenge mit dem Karbamatstrom in die Harnstoff-Synthese eingetragen, wodurch das Gleichgewicht der Harnstoff-Bildungsreaktion ungünstig beeinflusst wird. Ohne zusätzlichen Wassereintrag liegt die Harnstoffausbeute im Idealfall bei ca. 45%. Jedes weitere eingetragene Wassermolekül verschlechtert die Ausbeute. Umfangreiche Information findet man in der Literatur, z.B. in S. Kawasumi, Equilibrium of the CO2-NH3-H2O-Urea System under High Temperature and Pressure. III. Effect of Water Added on Vapor-Liquid Equilibrium, Bull. Chem. Soc. Jap, Vol 26 (1953), No. 5, pp. 218-222.

DE 32 39 605 A1 offenbart ein Verfahren zur kombinierten Herstellung von Ammoniak und Harnstoff, bei dem das im Wesentlichen aus Wasserstoff, Stickstoff und Kohlendioxid bestehende Ammoniaksynthesegas einer Druckwäsche bei Temperaturen von höchstens Umgebungstemperatur zur Entfernung saurer Verunreinigung, insbesondere Kohlendioxid, mit einem physikalisch wirkenden Lösungsmittel unterworfen wird, woraufhin das beladene Lösungsmittel zur Ausgasung von Inerten teilentspannt und anschließend drucklos regeneriert und zur Druckwäsche zurückgeführt wird und das bei der Regenerierung freiwerdende Kohlendioxid zur Harnstoffsynthese herangezogen wird. Dieses Verfahren ermöglicht jedoch nur eine geringfügige Reduzierung der Kompressionsarbeit. Zusätzlich wird in diesem Verfahren nur ein Teil des Kohlendioxids bei einem höheren Druck aus dem Lösungsmittel freigesetzt. Ein CO₂-Kompressor ist daher auch bei diesem Verfahren weiterhin erforderlich.

Die bekannten vollintegrierten Verfahren und Vorrichtungen zur Herstellung von Harnstoff sind daher nicht in jedem Punkt zufriedenstellend und es besteht ein Bedarf an verbesserten Verfahren und Vorrichtungen. Bisher ist keine produzierende vollintegrierte Anlage mit einer nennenswerten Leistung von beispielsweise 1000 Tagestonnen bekannt.

Diese Aufgabe wird durch den Gegenstand der Patentansprüche und die Beschreibung gelöst.

Ein erster Aspekt der Erfindung betrifft ein Verfahren zur Bereitstellung von CO₂ für die Harnstoffsynthese und zur Herstellung von Harnstoff, wobei das Verfahren die folgenden Schritte umfasst:
a) Bereitstellen eines Gasstroms A umfassend Wasserstoff, Stickstoff und Kohlendioxid;
b) Abtrennen zumindest eines Teils des Kohlendioxids des Gasstroms A mit einem Lösungsmittel unter Bildung eines Kohlendioxid-abgereicherten Gasstroms B und eines mit Kohlendioxid beladenen Lösungsmittels;
c) Synthetisieren von Ammoniak aus zumindest einem Teil des Wasserstoffs und aus zumindest einem Teil des Stickstoffs, welche in dem Gasstrom B enthalten sind;
d) Desorption des Kohlendioxids aus dem beladenen Lösungsmittel aus Schritt (b); und
e) Synthetisieren von Harnstoff aus zumindest einem Teil des in Schritt (c) synthetisierten Ammoniaks und aus zumindest einem Teil des in Schritt (d) desorbierten Kohlendioxids;
wobei die Desorption des Kohlendioxids in Schritt (d) bei einem höheren Druck erfolgt als das Synthetisieren des Harnstoffs in Schritt (e).

In Schritt (a) des erfindungsgemäßen Verfahrens wird ein Gasstrom A bereitgestellt, welcher hauptsächlich Wasserstoff, Stickstoff und Kohlendioxid umfasst. Ggf. kann der Gasstrom A weitere, bevorzugt inerte Komponenten wie Methan, Argon, Kohlenmonoxid und/oder Helium umfassen. Der Gasstrom A wird bevorzugt als Synthesegas aus Kohlenwasserstoffen, vorzugsweise aus Erdgas, Wasser in Form von Dampf und Luft bzw. Sauerstoff durch Reformierung und anschließende Gasreinigung gewonnen. Geeignete Verfahren zur Erzeugung eines solchen Synthesegases sind einem Fachmann bekannt und es kann diesbezüglich beispielsweise vollumfänglich verwiesen werden auf A. Nielsen, I. Dybkjaer, Ammonia - Catalysis and Manufacture, Springer Berlin 1995, Kapitel 6, Seiten 202-326; M. Appl, Ammonia. Principles and Industrial Practice, WILEY-VCH Verlag GmbH 1999. In einer bevorzugten Ausführungsform wird zumindest ein Teil des Gasstroms A durch Dampfreformierung und/oder durch autotherme Reformierung bereitgestellt.

Der in Schritt (a) des erfindungsgemäßen Verfahrens bereitgestellte Gasstrom A kann als Synthesegas bereits herkömmlichen Aufbereitungsmaßnahmen unterzogen worden sein, wie z.B. Heliumentfernung, Erdgasentschwefelung und/oder Konvertierung von Kohlenmonoxid zu Kohlendioxid.

In Schritt (b) des erfindungsgemäßen Verfahrens wird zumindest ein Teil des Kohlendioxids des Gasstroms A mit einem Lösungsmittel unter Bildung eines Kohlendioxid-abgereicherten Gasstroms B und eines mit Kohlendioxid beladenen Lösungsmittels abgetrennt. Dabei muss der Gasstrom ggf. abgekühlt werden auf Temperaturen von 30 - 70 °C.

In einer bevorzugten Ausführungsform umfasst das Lösungsmittel, mit welchem das Kohlendioxid in Schritt (b) abgetrennt wird, ein Ammoniak/Wasser-Gemisch. Bevorzugt liegt der Anteil an Ammoniak in dem Ammoniak/Wasser-Gemisch im Bereich von 1 bis 50 Gew.-%, bevorzugter im Bereich von 5 bis 45 Gew.-%, im Bereich von 10 bis 40 Gew.-%, im Bereich von 15 bis 35 Gew.-%, im Bereich von 20 bis 30 Gew.-% und besonders bevorzugt im Bereich von 20 bis 25 Gew.-%. In einem Ammoniak/Wasser-Gemisch wird das Kohlendioxid bevorzugt chemisch in Form von Karbamat- und/oder Karbonat-Ionen gebunden.

In Schritt (c) des erfindungsgemäßen Verfahrens wird Ammoniak aus zumindest einem Teil des Wasserstoffs und aus zumindest einem Teil des Stickstoffs, welche in dem Gasstrom B enthalten sind, synthetisiert. Dazu wird der Gasstrom B in einen Ammoniakreaktor geleitet. Ggf. umfasst der Gasstrom B noch Reste von Kohlendioxid und/oder Kohlenmonoxid, welche bevorzugt vor der Synthese von Ammoniak beispielsweise durch kryogene Methoden oder durch eine Druckwechsel-Adsorption von dem Gasstrom B abgetrennt werden. Bevorzugt werden diese Reste durch Methanisierung zu Methan umgesetzt. Geeignete Verfahren zur Hydrierung von Kohlenmonoxid und Kohlendioxid zu Methan sind einem Fachmann bekannt. Durch die Methanisierung wird der Gehalt an Kohlenmonoxid, und dabei auch ggf. Kohlendioxid, in dem Gasstrom B verringert und der Gehalt an Methan in dem Gasstrom erhöht. Ggf. wird vor der Ammoniaksynthese der Gasstrom B auf einen erhöhten Druck verdichtet, vorzugsweise auf einen Druck im Bereich von 120 bis 250 bar, bevorzugter im Bereich von 180 bis 220 bar. Vorzugsweise weist der Ammoniakreaktor zumindest ein Katalysatorbett auf, welches von dem Gasstrom B nicht rein axial, sondern vorwiegend radial durchströmt wird, vorzugsweise von außen nach innen. Bevorzugt wird danach zumindest ein Teil des im Ammoniakreaktor gebildeten Ammoniaks von dem resultierenden Produktstrom durch Abkühlen abgetrennt, wozu der Gasstrom bevorzugt zunächst einen Wärmetauscher und anschließend eine Kondensationsvorrichtung durchläuft. Dabei wird der Gasstrom abgekühlt, bevorzugt auf Temperaturen im Bereich von -15°C bis -79°, bevorzugter im Bereich von -25°C bis -79°C, im Bereich von -35°C bis -79°C oder im Bereich von -35°C bis -50°C, so dass Ammoniak unter den gegebenen Bedingungen auskondensiert und so von der Gasphase durch Phasentrennung abgetrennt werden kann.

In einer bevorzugten Ausführungsform wird das mit Kohlendioxid beladene Lösungsmittel nach Schritt (b) und vor Schritt (d) zumindest teilweise entspannt, um in Schritt (b) koabsorbierte Komponenten wie beispielsweise Wasserstoff und Stickstoff aus dem Lösungsmittel zu entfernen. Dazu wird das mit Kohlendioxid beladene Lösungsmittel nach Schritt (b) über eine Zwischenentspannungsstufe (Flash) zum Entfernen von Inerten geführt. Bei der Entspannung des beladenen Lösungsmittels kann man eine Hydroturbine für die Rückgewinnung von Energie einsetzen. Bevorzugt wird das mit Kohlendioxid beladene Lösungsmittel nach Schritt (b) und vor Schritt (d) auf einen Druck unterhalb von 10 bar entspannt. Diese Lösung wird großtechnisch an vergleichbaren Stellen eingesetzt und ist einem Fachmann bekannt.

Die entspannte, noch mit CO₂ beladene Lösung wird mit Hilfe einer Pumpe komprimiert. Der Zieldruck soll dabei bevorzugt oberhalb des Harnstoff-Synthesedruckes sein und ausreichend hoch sein, um die Druckverluste in dem Wärmetauscher, in der Desorptionskolonne und in den Rohrleitungen zu kompensieren. In der Praxis werden die Druckverluste normalerweise die Werte von maximal 30 bar nicht übersteigen. In einer anderen bevorzugten Ausführungsform soll der Zieldruck dabei mindestens so weit oberhalb des Harnstoff-Synthesedruckes liegen, dass das CO₂ trotz der Druckverluste in den zwischen der Pumpe und der Harnstoffsynthese im Strömungsweg liegenden Rohrleitungen, Regelventile und Apparate bevorzugt ohne eine weitere Verdichtung in die Harnstoffsynthese eingebracht werden kann. Bevorzugt liegt der Zieldruck mindestens 1 bar oberhalb des Harnstoff-Synthesedrucks, bevorzugter mindestens 2 bar, mindestens 4 bar, mindestens 6 bar, mindestens 8 bar, mindestens 10 bar, mindestens 20 bar oder mindestens 30 bar. In einer anderen bevorzugten Ausführungsform liegt der Zieldruck so weit oberhalb des Harnstoff-Synthesedruckes, dass die Druckdifferenz zwischen Zieldruck und dem Harnstoff-Synthesedruck im Bereich von 1 bis 40 bar liegt, bevorzugter im Bereich von 2 bis 30 bar oder im Bereich von 5 bis 20 bar.

In Schritt (d) des erfindungsgemäßen Verfahrens erfolgt die Desorption des Kohlendioxids aus dem Lösungsmittel in einer geeigneten Desorptionsvorrichtung, bevorzugt in einer Desorptionskolonne, welche bevorzugt als Rektifikationskolonne ausgeführt ist. Bevorzugt erfolgt die Desorption durch Erwärmen des Lösungsmittels, welches bevorzugt einen höheren Siedepunkt aufweist als das Kohlendioxid. Bevorzugt erfolgt die Desorption des Kohlendioxids bei einem höheren Druck als das Synthetisieren des Harnstoffs in Schritt (e) und die Druckdifferenz zwischen dem Druck, bei dem die Desorption erfolgt, und dem Druck, bei dem in Schritt (e) Harnstoff synthetisiert wird, ist ausreichend hoch, um die ggf. auftretenden Druckverluste des Kohlendioxids auf dem Weg in die Harnstoffsynthese zu kompensieren. Bevorzugt ist der Druck, bei dem die Desorption des Kohlendioxids erfolgt, mindestens 1 bar höher als der Druck, bei dem der Harnstoff synthetisiert wird, bevorzugter mindestens 2 bar, mindestens 4 bar, mindestens 6 bar, mindestens 8 bar, mindestens 10 bar, mindestens 20 bar oder mindestens 30 bar. In einer anderen bevorzugten Ausführungsform erfolgt die Desorption des Kohlendioxids bei einem Druck, welcher oberhalb des Harnstoff-Synthesedrucks liegt, so dass die Druckdifferenz zwischen der Desorption des Kohlendioxids und dem Harnstoff-Synthesedruck im Bereich von 1 bis 40 bar liegt, bevorzugter im Bereich von 2 bis 30 bar oder im Bereich von 5 bis 20 bar. Bevorzugt wird die Temperatur, bei der die Desorption in Schritt (d) durchgeführt wird, so gewählt, dass das Kohlendioxid bei dem jeweils vorherrschenden Druck desorbieren kann. Die Temperatur ergibt sich aus dem konkreten Harnstoff-Synthesedruck und dem notwendigen Überdruck für die Überwindung der Druckverluste. Typischerweise liegt der Harnstoffsynthesedruck bei mindestens 120 bar bis 150 bar und der Überdruck beträgt bevorzugt mindestens 1 bar, bevorzugter mindestens 2 bar, mindestens 3 bar, mindestens 4 bar, mindestens 5 bar, mindestens 6 bar, mindestens 8 bar, mindestens 10 bar, mindestens 20 bar oder mindestens 30 bar. In einer bevorzugten Ausführungsform erfolgt die Desorption des Kohlendioxids bei einer Temperatur im Bereich von 100°C bis 300°C, bevorzugter im Bereich von 130°C bis 270°C, im Bereich von 150°C bis 250°C oder im Bereich von 170 bis 230 °C.

In Schritt (e) des erfindungsgemäßen Verfahrens wird Harnstoff aus zumindest einem Teil des in Schritt (c) synthetisierten Ammoniaks und aus zumindest einem Teil des in Schritt (d) desorbierten Kohlendioxids synthetisiert. Dazu wird der in Schritt (c) synthetisierte Ammoniak zunächst bevorzugt verdichtet, bevorzugt auf einen Druck von mindestens 100 bar, bevorzugter auf einen Druck von mindestens 120 bar, mindestens 140 bar oder mindestens 150 bar.

Bevorzugt erfolgt die Synthese von Harnstoff bei einem Druck im Bereich von 100 bis 300 bar, bevorzugter im Bereich von 120 bis 200 bar oder im Bereich von 140 bis 160 bar. Das in Schritt (e) hergestellte Produkt umfasst im Wesentlichen Harnstoff, Wasser und Ammoniumkarbamat sowie ggf. nicht reagiertes Ammoniak und Kohlendioxid. Das Ammoniumkarbamat und der Ammoniak sowie das Kohlendioxid müssen aus der Lösung entfernt werden und können danach erneut für das Reaktionsverfahren zur Harnstoffsynthese bereitgestellt werden. Das Entfernen wird üblicherweise durch Strippen mit CO₂ oder NH₃ erreicht. Diese Verfahren sind Stand der Technik bei Harnstoffanlagen und dem Fachmann bekannt. Die Synthese von Harnstoff erfolgt bevorzugt in einer herkömmlichen Harnstoffanlage, deren Aufbau einem Fachmann bekannt ist.

Die Desorption des Kohlendioxids in Schritt (d) erfolgt bei einem höheren Druck als das Synthetisieren des Harnstoffs in Schritt (e). Bevorzugt erfolgt die Desorption des Kohlendioxids in Schritt (d) bei einem Druck, welcher mindestens 1 bar höher ist als der Druck, bei dem der Harnstoff synthetisiert wird, bevorzugter mindestens 2 bar, mindestens 3 bar, mindestens 4 bar, mindestens 5 bar, mindestens 6 bar, mindestens 10 bar, mindestens 20 bar, mindestens 30 bar, bevorzugt bei einem Druck von mindestens 120 bar, bevorzugter von mindestens 140 bar. In einer anderen bevorzugten Ausführungsform erfolgt die Desorption des Kohlendioxids bei einem Druck, welcher oberhalb des Harnstoff-Synthesedrucks liegt, so dass die Druckdifferenz zwischen dem Druck, bei dem die Desorption des Kohlendioxids erfolgt und dem Harnstoff-Synthesedruck im Bereich von 1 bis 40 bar liegt, bevorzugter im Bereich von 2 bis 30 bar oder im Bereich von 5 bis 20 bar. Bevorzugt kann das Kohlendioxid nach der Desorption in Schritt (d) ohne weitere Verdichtung direkt der Harnstoffsynthese in Schritt (e) zugeführt werden.

Es wurde überraschend gefunden, dass das Kohlendioxid mit der erforderlichen Reinheit mit einem noch moderaten Temperaturniveau aus dem Lösungsmittel desorbiert werden kann. Das benötigte Temperaturniveau ist aber mit etwa 200°C so hoch, dass das Konzept mit herkömmlichen Lösungsmitteln wegen ihrer begrenzten thermischen Stabilität nicht realisiert werden könnte. Wegen des relativ hohen Dampfdrucks bereits bei mäßigen Temperaturen und der verhältnismäßig großen Desorptionswärme ist der Einsatz von Ammoniak in dieser Form als reines Lösungsmittel in einer Gaswäsche daher nicht naheliegend. Zusätzlich wird bei dem erfindungsgemäßen Verfahren im Gegensatz zu bekannten Offenbarungen (vgl. z.B. DE 1 668 547 oder DE 26 13 102 C2) ein zusätzlicher Wassereintrag mit dem Kohlendioxid in die Harnstoffsynthese weitgehend vermieden.

In einer bevorzugten Ausführungsform wird die zur Desorption des Kohlendioxids in Schritt (d) erforderliche Energie durch Dampf bereitgestellt, welcher z.B. beim Synthetisieren von Ammoniak erzeugt wird. In einer bevorzugten Ausführungsform wird der Dampf, der bei der Desorption des Kohlendioxids zum Einsatz kommen soll, mit der beim Synthetisieren von Ammoniak freiwerdenden Prozesswärme erzeugt. Bevorzugt wird dabei der Dampf über einen Wärmetauscher geleitet und überträgt dadurch einen Teil der zur Desorption erforderlichen Energie auf das Kohlendioxid-haltige Lösungsmittel.

Es wurde überraschend festgestellt, dass die für die Desorption des Kohlendioxids in Schritt (d) und die für das Abtrennen des Kohlendioxids in Schritt (b) benötigte Wärmemenge mit begrenzten Modifikationen des Dampfsystems in kombinierten Ammoniak-Harnstoff-Anlagen verfügbar gemacht werden kann. Da die Desorption des Kohlendioxids in Schritt (d) bei einem höheren Druck erfolgt als das Synthetisieren des Harnstoffs in Schritt (e), kann das nach der Desorption freigesetzte Kohlendioxid ohne eine weitere Verdichtung zum Synthetisieren des Harnstoffs eingesetzt werden, wodurch ein Kohlendioxid-Verdichter eingespart werden kann. Da der Dampfbedarf für den Antrieb des Kohlendioxid-Verdichters entfällt, ergibt sich insgesamt eine signifikante Reduzierung des Energiebedarfs.

Mit den Annahmen, dass
- die Harnstoff-Anlage weitgehend unverändert bleibt,
- das Abtrennen des Kohlendioxids mit Ammoniak oder Ammoniak/Wasser-Lösungen einen ähnlichen apparativen Aufwand aufweist wie die derzeit dem Stand der Technik entsprechenden und in aktuell errichteten Ammoniak-Anlagen eingesetzten Kohlendioxid-Wäschen und
- die Modifikationen des Dampfsystems weitgehend kostenneutral sind
ergibt sich durch den Wegfall des Kohlendioxid-Verdichters einschließlich seiner Zusatzeinrichtungen (Antriebsturbine, Zwischenkühler, Ölsystem etc.) eine substanzielle Reduzierung der Anlageninvestitionskosten.

Ein separater Betrieb der Ammoniak-Anlage ohne Harnstoff-Anlage ist auch bei diesem Konzept möglich, da das abgetrennte Kohlendioxid mit minimalem Aufwand entspannt und wie bei der konventionellen Prozessführung dann an die Umgebung abgegeben werden kann. Lediglich ein Alleinbetrieb der Harnstoff-Anlage wäre ohne zusätzliche Einrichtungen zur Bereitstellung des Kohlendioxids auf Harnstoff-Synthesedruckniveau nicht mehr ohne weiteres realisierbar.

In einer bevorzugten Ausführungsform umfasst das Synthetisieren des Harnstoffs in Schritt (e) die Teilreaktionen (i) und (ii):
(i) Bildung von Ammoniumkarbamat aus Ammoniak und Kohlendioxid; und
(ii) Umsetzen des Ammoniumkarbamats zu Harnstoff und Wasser;
wobei die Energie für die endotherme Teilreaktion (ii) zumindest teilweise aus der exothermen Teilreaktion (i) gedeckt wird.

Die beiden Teilreaktionen werden wegen der Baugröße der Apparate bevorzugt aber nicht zwingend jeweils in separaten Reaktionsvorrichtungen durchgeführt, welche bevorzugt in unmittelbarer Nachbarschaft zueinander angeordnet sind, so dass die bei der Bildung von Ammoniumkarbamat aus Ammoniak und Kohlendioxid freigesetzte Wärme weitestgehend ohne Verlust zur Dehydratation des Ammoniumkarbamats verwendet werden kann. Auf diesem Wege kann eine energetisch günstige Gesamtlösung realisiert werden, da die Reaktionswärme der Karbamatbildungsreaktion für die Harnstoffbildung genutzt wird und nicht über Kühlwasser ungenutzt abgeführt wird.

Ein weiterer Aspekt der Erfindung betrifft die Verwendung einer Vorrichtung zur Herstellung von Harnstoff, wobei die Vorrichtung die folgenden miteinander in Wirkverbindung stehenden Komponenten umfasst:
(A) Vorrichtung zur Bereitstellung eines Gasstroms umfassend Wasserstoff, Stickstoff und Kohlendioxid;
(B) Mittel zum Abtrennen zumindest eines Teils des Kohlendioxids des Gasstroms mit einem Lösungsmittel;
(C) Ammoniaksyntheseeinheit, umfassend einen Ammoniakreaktor zum Synthetisieren von Ammoniak aus zumindest einem Teil des Wasserstoffs und aus zumindest einem Teil des Stickstoffs, welche in dem Gasstrom enthalten sind;
(D) Mittel zum Desorbieren des Kohlendioxids aus dem Lösungsmittel; und
(E) Harnstoffsyntheseeinheit, umfassend einen Harnstoffreaktor zum Synthetisieren von Harnstoff aus zumindest einem Teil des in der Ammoniaksyntheseeinheit synthetisieren Ammoniaks und aus zumindest einem Teil des aus dem Lösungsmittel desorbierten Kohlendioxids;
wobei die Mittel zum Desorbieren des Kohlendioxids bei einem höheren Druck betrieben werden als die Harnstoffsyntheseeinheit.

Alle im Zusammenhang mit dem erfindungsgemäßen Verfahren beschriebenen bevorzugten Ausführungsformen gelten analog für die erfindungsgemäße Verwendung einer Vorrichtung und werden daher an dieser Stelle nicht wiederholt.

Die erfindungsgemäße Verwendung einer Vorrichtung umfasst eine Vorrichtung zur Bereitstellung eines Gasstroms umfassend Wasserstoff, Stickstoff, Kohlendioxid und ggf. weitere inerte Komponenten. Bevorzugt umfasst die Vorrichtung zur Bereitstellung eines Gasstroms einen Dampfreformer und/oder einen autothermen Reformer. Umfasst die Vorrichtung einen autothermen Reformer, kann die Menge an Kohlendioxid, welche in dem autothermen Reformer gebildet wird, durch Variation von Reaktionsparametern wie beispielsweise dem Druck oder dem Dampf/Kohlenstoff-Verhältnis kontrolliert werden. Bevorzugt wird im autothermen Reformer gerade so viel Kohlendioxid gebildet, dass das in der Vorrichtung zur Herstellung von Ammoniak hergestellte Ammoniak bevorzugt vollständig mit Kohlendioxid zu Harnstoff umgesetzt werden kann.

Weiterhin umfasst die erfindungsgemäße Verwendung einer Vorrichtung Mittel zum Abtrennen zumindest eines Teils des Kohlendioxids mit einem Lösungsmittel. Bevorzugt umfassen die Mittel zum Abtrennen zumindest eines Teils des Kohlendioxids eine Ammoniak-Wasser-Wäsche.

Weiterhin umfasst die erfindungsgemäße Verwendung einer Vorrichtung eine Ammoniaksyntheseeinheit, umfassend einen Ammoniakreaktor zum Synthetisieren von Ammoniak aus zumindest einem Teil des Wasserstoffs und aus zumindest einem Teil des Stickstoffs, welche in dem Gasstrom enthalten sind. Bevorzugt umfasst der Ammoniakreaktor mindestens ein Katalysatorbett, welches von dem Gasstrom bevorzugt nicht rein axial, sondern vorwiegend radial durchströmt wird, vorzugsweise von außen nach innen.

Darüber hinaus umfasst die erfindungsgemäße Verwendung einer Vorrichtung Mittel zum Desorbieren des Kohlendioxids aus dem Lösungsmittel. Beispielsweise können die Mittel zum Desorbieren eine Destillationskolonne umfassen. Bevorzugt werden die Mittel zum Desorbieren des Kohlendioxids, insbesondere die Destillationskolonne, bei einem Druck betrieben, welcher oberhalb des Druckes liegt, bei dem die Harnstoffsyntheseeinheit betrieben wird und ausreichend hoch ist, um die Druckverluste des Kohlendioxids bis zum Eintritt in die Harnstoffsynthese zu überwinden. Bevorzugt ist der Druck, bei dem die Mittel zum Desorbieren des Kohlendioxids betrieben werden,mindestens 1 bar höher als der Druck, bei dem die Harnstoffsyntheseeinheit betrieben wird, bevorzugter mindestens 2 bar, mindestens 3 bar, mindestens 4 bar, mindestens 5 bar, mindestens 6 bar, mindestens 8 bar mindestens 10 bar, mindestens 20 bar oder mindestens 30 bar. Bevorzugt werden die Mittel zum Desorbieren des Kohlendioxids bei einem Druck von mindestens 120 bar betrieben, bevorzugter von mindestens 150 bar. In einer anderen bevorzugten Ausführungsform werden die Mittel zum Desorbieren des Kohlendioxids bei einem höheren Druck betrieben als die Harnstoffsyntheseeinheit, so dass die Druckdifferenz zwischen dem Druck, bei dem die Mittel zum Desorbieren des Kohlendioxids betrieben werden und dem Druck, bei dem die Harnstoffsyntheseeinheit betrieben wird, im Bereich von 1 bis 40 bar liegt, bevorzugter im Bereich von 2 bis 30 bar oder im Bereich von 5 bis 20 bar.

Die erfindungsgemäße Verwendung einer Vorrichtung umfasst weiterhin eine Harnstoffsyntheseeinheit. Bevorzugt umfasst die Harnstoffsyntheseeinheit zwei voneinander räumlich getrennte Reaktionskammern, wobei in einer ersten Reaktionskammer bevorzugt die Umsetzung von Kohlendioxid und Ammoniak zu Ammoniumkarbamat und in einer zweiten Reaktionskammer bevorzugt die Dehydratation des Ammoniumkarbamats stattfindet.

Bevorzugt werden die Mittel zum Desorbieren des Kohlendioxids bei einem Druck betrieben, welcher oberhalb des Druckes liegt, bei dem die Harnstoffsyntheseeinheit betrieben wird und die Druckdifferenz zwischen dem Druck, bei dem die Mittel zum Desorbieren des Kohlendioxids und dem Druck, bei dem die Harnstoffsyntheseeinheit betrieben werden, ist ausreichend hoch, um die ggf. auftretenden Druckverluste des Kohlendioxids bis zum Eintritt in die Harnstoffsyntheseeinheit zu kompensieren. Bevorzugt werden die Mittel zum Desorbieren des Kohlendioxids bei einem Druck betrieben, welcher mindestens 1 bar oberhalb des Drucks liegt, bei dem die Harnstoffsyntheseeinheit betrieben wird, bevorzugter mindestens 2 bar, mindestens 3 bar, mindestens 4 bar, mindestens 5 bar, mindestens 6 bar, mindestens 8 bar, mindestens 10 bar, mindestens 20 bar oder mindestens 30 bar. In einer anderen bevorzugten Ausführungsform werden die Mittel zum Desorbieren des Kohlendioxids bei einem höheren Druck betrieben als die Harnstoffsyntheseeinheit, so dass die Druckdifferenz zwischen dem Druck, bei dem die Mittel zum Desorbieren des Kohlendioxids betrieben werden und dem Druck, bei dem die Harnstoffsyntheseeinheit betrieben wird, im Bereich von 1 bis 40 bar liegt, bevorzugter im Bereich von 2 bis 30 bar oder im Bereich von 5 bis 20 bar. Dementsprechend sind bevorzugt zwischen den Mitteln zum Desorbieren des Kohlendioxids und der Harnstoffsyntheseeinheit keine weiteren Mittel zum Verdichten des Kohlendioxids angeordnet.

Die erfindungsgemäße Verwendung einer Vorrichtung eignet sich besonders zum Durchführen des erfindungsgemäßen Verfahrens.

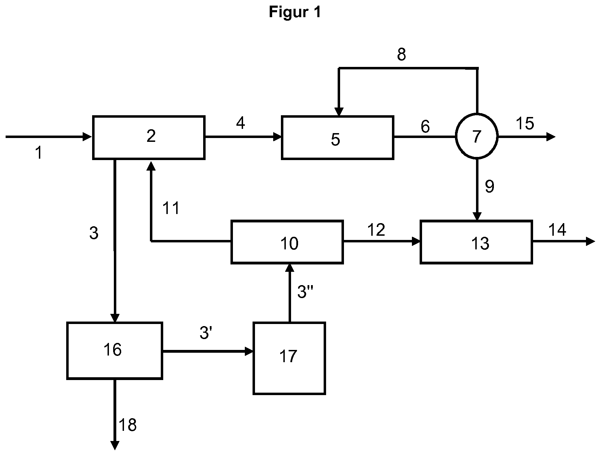

Figur 1 zeigt beispielhaft die Strömungsverläufe in der erfindungsgemäßen Verwendung einer Vorrichtung.

Ein Gasstrom A (1), welcher Wasserstoff, Stickstoff, Kohlenmonoxid, Kohlendioxid und ggf. weitere, bzgl. der Ammoniaksyntheseeinheit inerte Komponenten wie Methan oder Argon umfasst, wird bevorzugt in einem Dampfreformer, ggf. einem autothermen Reformer bereitgestellt. Zumindest ein Teil des im Gasstrom A (1) befindlichen Kohlendioxids wird in einer Abtrennvorrichtung (2) bevorzugt durch eine Ammoniak-Wasser-Wäsche abgetrennt. Der dabei gebildete, Kohlendioxid-abgereicherte Gasstrom B (4) wird einer Ammoniaksyntheseeinheit (5) zugeleitet, in der zumindest ein Teil des Wasserstoffs und zumindest ein Teil des Stickstoffs des Gasstroms B (4) zu Ammoniak umgesetzt werden. Aus der Ammoniaksyntheseeinheit (5) wird ein Produktstrom (6) ausgeleitet, welcher überwiegend Ammoniak, Wasserstoff und Stickstoff umfasst. Zumindest ein Teil des Ammoniaks des Produktstroms (6) wird in einer Ammoniakabtrennvorrichtung (7) abgetrennt und als Ammoniakstrom (9) zur Harnstoffsyntheseeinheit (13) geleitet. Ggf. kann zumindest ein Teil des Ammoniaks nach dem Ausleiten aus der Ammoniakabtrennvorrichtung (7) verdichtet werden. Der Ammoniak-abgereicherte Produktstrom (8) wird zur Ammoniaksyntheseeinheit (5) zurückgeführt. Ein ggf. vorhandener Ammoniak-Überschuss-Strom (15) kann einer weiteren Verwendung zugeführt werden, wie es dem Stand der Technik entspricht.

Das die Abtrennvorrichtung (2) verlassende Kohlendioxid-angereicherte Lösungsmittel (3) wird ggf. zunächst einer Zwischenentspannungsstufe (16) zugeführt, in der ggf. in der Abtrennvorrichtung (2) koabsorbierte Komponenten (18) aus dem Lösungsmittel entfernt werden. Das Kohlendioxid-angereicherte Lösungsmittel (3') wird bevorzugt mit einer Pumpe (17) komprimiert, wobei der Zieldruck bevorzugt 1 bar bis 30 bar oberhalb des Harnstoffsynthesedrucks liegt. Anschließend wird das Kohlendioxid-angereicherte und komprimierte Lösungsmittel (3") einer Desorptionsvorrichtung (10) zugeführt, in welcher das Kohlendioxid (12) aus dem Lösungsmittelstrom (3") desorbiert wird, bevorzugt bei einem Druck, welcher oberhalb des Betriebsdrucks der Harnstoffsyntheseeinheit (13) liegt. Der regenerierte Lösungsmittelstrom (11), welcher aus der Desorptionsvorrichtung (10) ausgeleitet wird, kann erneut in der Abtrennvorrichtung (2) verwendet werden. Zumindest ein Teil des Kohlendioxidstroms (12), welcher in der Desorptionsvorrichtung (10) desorbiert wird, wird in die Harnstoffsyntheseeinheit (13) geleitet und dort mit dem Ammoniakstrom (9) unter Bildung von Harnstoff umgesetzt. Der Harnstoffstrom (14) verlässt die Harnstoffsyntheseeinheit (13) und wird ggf. weiter verarbeitet.

### Bezugszeichenliste:

- 1.: Gasstrom A
- 2.: Abtrennvorrichtung
- 3, 3', 3".: Kohlendioxid-angereichertes Lösungsmittel
- 4.: Gasstrom B
- 5.: Ammoniaksyntheseeinheit
- 6.: Produktstrom
- 7.: Ammoniakabtrennvorrichtung
- 8.: Ammoniak-abgereicherter Produktstrom
- 9.: Ammoniakstrom
- 10.: Desorptionsvorrichtung
- 11.: Lösungsmittelstrom regeneriert
- 12.: Kohlendioxidstrom
- 13.: Harnstoffsyntheseeinheit
- 14.: Harnstoffstrom
- 15.: Ammoniak-Überschussstrom
- 16.: Zwischenentspannungsstufe
- 17.: Pumpe
- 18: koabsorbierte Komponenten

## Patentansprüche

1. Verfahren zur Herstellung von Harnstoff, wobei das Verfahren die folgenden Schritte umfasst:
a) Bereitstellen eines Gasstroms A (1) umfassend Wasserstoff, Stickstoff und Kohlendioxid;
b) Abtrennen zumindest eines Teils des Kohlendioxids des Gasstroms A mit einem Lösungsmittel unter Bildung eines Kohlendioxid-abgereicherten Gasstroms B (4);
c) Synthetisieren von Ammoniak aus zumindest einem Teil des Wasserstoffs und aus zumindest einem Teil des Stickstoffs, welche in dem Gasstrom B (4) enthalten sind;
d) Desorption des Kohlendioxids (12) aus dem Lösungsmittel (3"); und
e) Synthetisieren von Harnstoff aus zumindest einem Teil des in Schritt (c) synthetisierten Ammoniaks und aus zumindest einem Teil des in Schritt (d) desorbierten Kohlendioxids;
wobei die Desorption des Kohlendioxids in Schritt (d) bei einem höheren Druck erfolgt als das Synthetisieren des Harnstoffs in Schritt (e).

2. Das Verfahren nach Anspruch 1, wobei das mit Kohlendioxid beladene Lösungsmittel nach Schritt (b) und vor Schritt (d) zumindest teilweise entspannt wird, um in Schritt (b) koabsorbierte Komponenten aus dem Lösungsmittel zu entfernen.

3. Das Verfahren gemäß einem der vorstehenden Ansprüche, wobei die Desorption des Kohlendioxids in Schritt (d) bei einem höheren Druck erfolgt als das Synthetisieren des Harnstoffs in Schritt (e) und die Druckdifferenz zwischen dem Druck, bei dem die Desorption erfolgt, und dem Druck, bei dem in Schritt (e) Harnstoff synthetisiert wird, ausreichend hoch ist, um ggf. auftretende Druckverluste des Kohlendioxids bis zum Eintritt in die Harnstoffsynthese zu kompensieren.

4. Das Verfahren gemäß einem der vorstehenden Ansprüche, wobei die Temperatur, bei der die Desorption in Schritt (d) durchgeführt wird, so gewählt wird, dass das Kohlendioxid bei dem jeweils vorherrschenden Druck desorbieren kann.

5. Das Verfahren gemäß einem der vorstehenden Ansprüche, wobei das Lösungsmittel, mit welchem das Kohlendioxid in Schritt (b) abgetrennt wird, ein Ammoniak/WasserGemisch umfasst.

6. Das Verfahren gemäß einem der vorstehenden Ansprüche, wobei die Desorption des Kohlendioxids in Schritt (d) bei einer Temperatur im Bereich von 130°C bis 270°C erfolgt.

7. Das Verfahren nach einem der vorstehenden Ansprüche, wobei die zur Desorption des Kohlendioxids in Schritt (d) erforderliche Energie durch Dampf bereitgestellt wird.

8. Das Verfahren nach Anspruch 7, wobei der Dampf, der bei der Desorption des Kohlendioxids zum Einsatz kommen soll, mit der beim Synthetisieren von Ammoniak freiwerdenden Prozesswärme erzeugt wird.

9. Das Verfahren nach einem der vorstehenden Ansprüche, wobei zumindest ein Teil des Gasstroms A durch Dampfreformierung und/oder durch autotherme Reformierung bereitgestellt wird.

10. Das Verfahren nach einem der vorstehenden Ansprüche, wobei das Synthetisieren des Harnstoffs in Schritt (e) die Teilreaktionen (i) und (ii) umfasst:
(i) Bildung von Ammoniumkarbamat aus Ammoniak und Kohlendioxid; und
(ii) Umsetzen des Ammoniumkarbamats zu Harnstoff und Wasser;
wobei die Energie für die endotherme Teilreaktion (ii) zumindest teilweise aus der exothermen Teilreaktion (i) gedeckt wird.

11. Verwendung einer Vorrichtung zur Herstellung von Harnstoff, wobei die Vorrichtung die folgenden miteinander in Wirkverbindung stehenden Komponenten umfasst:
(A) Vorrichtung zur Bereitstellung eines Gasstroms umfassend Wasserstoff, Stickstoff und Kohlendioxid;
(B) Mittel zum Abtrennen (2) zumindest eines Teils des Kohlendioxids des Gasstroms mit einem Lösungsmittel;
(C) Ammoniaksyntheseeinheit (5), umfassend einen Ammoniakreaktor zum Synthetisieren von Ammoniak aus zumindest einem Teil des Wasserstoffs und aus zumindest einem Teil des Stickstoffs, welche in den Gasströmen (1) und (4) enthalten sind;
(D) Mittel zum Desorbieren des Kohlendioxids (10) aus dem Lösungsmittel; und
(E) Harnstoffsyntheseeinheit (13), umfassend einen Harnstoffreaktor zum Synthetisieren von Harnstoff aus zumindest einem Teil des in der Ammoniaksyntheseeinheit (5) synthetisieren Ammoniaks und aus zumindest einem Teil des aus dem Lösungsmittel desorbierten Kohlendioxids;
wobei die Mittel zum Desorbieren des Kohlendioxids (10) bei einem höheren Druck betrieben werden als die Harnstoffsyntheseeinheit (13), in einem Verfahren gemäß einem der Ansprüche 1 bis 10.

12. Verwendung gemäß Anspruch 11, wobei die Mittel zum Desorbieren des Kohlendioxids (10) bei einem Druck betrieben werden, welcher oberhalb des Drucks liegt, bei dem die Harnstoffsyntheseeinheit betrieben wird und die Druckdifferenz zwischen dem Druck, bei dem die Mittel zum Desorbieren des Kohlendioxids und dem Druck, bei dem die Harnstoffsyntheseeinheit betrieben werden, ausreichend hoch ist, um die ggf. auftretenden Druckverluste des Kohlendioxids bis zum Eintritt in die Harnstoffsyntheseeinheit zu kompensieren.

13. Verwendung gemäß einem der Ansprüche 11 oder 12, wobei die Vorrichtung zur Bereitstellung eines Gasstroms einen Dampfreformer und/oder einen autothermen Reformer umfasst.

14. Verwendung gemäß einem der Ansprüche 11 bis 13, wobei die Mittel zum Abtrennen zumindest eines Teils des Kohlendioxids (2) eine Ammoniak-Wasser-Wäsche umfassen.

## Claims

1. Process for preparing urea, wherein the process comprises the following steps:
a) provision of a gas stream A (1) comprising hydrogen, nitrogen and carbon dioxide;
b) removal of at least part of the carbon dioxide of the gas stream A by means of a solvent to form a carbon dioxide-depleted gas stream B (4) ;
c) synthesis of ammonia from at least part of the hydrogen and at least part of the nitrogen which are present in the gas stream B (4);
d) desorption of the carbon dioxide (12) from the solvent (3"); and
e) synthesis of urea from at least part of the ammonia synthesized in step (c) and at least part of the carbon dioxide desorbed in step (d) ;
where the desorption of the carbon dioxide in step (d) is carried out at a higher pressure than the synthesis of the urea in step (e).

2. Process according to Claim 1, wherein the solvent loaded with carbon dioxide is at least partially depressurized after step (b) and before step (d) in order to remove components which have been coabsorbed in step (b) from the solvent.

3. Process according to either of the preceding claims, wherein the desorption of the carbon dioxide in step (d) is carried out at a higher pressure than the synthesis of the urea in step (e) and the pressure difference between the pressure at which desorption is carried out and the pressure at which urea is synthesized in step (e) is sufficiently high to compensate for any pressure drops of the carbon dioxide occurring before entry into the urea synthesis.

4. Process according to any of the preceding claims, wherein the temperature at which the desorption in step (d) is carried out is selected so that the carbon dioxide can desorb at the pressure prevailing in each case.

5. Process according to any of the preceding claims, wherein the solvent by means of which the carbon dioxide is separated off in step (b) comprises an ammonia/water mixture.

6. Process according to any of the preceding claims, wherein the desorption of the carbon dioxide in step (d) is carried out at a temperature in the range from 130°C to 270°C.

7. Process according to any of the preceding claims, wherein the energy required for desorption of the carbon dioxide in step (d) is provided by steam.

8. Process according to Claim 7, wherein the steam which is to be used in the desorption of the carbon dioxide is produced by means of the process heat liberated in the synthesis of ammonia.

9. Process according to any of the preceding claims, wherein at least part of the gas stream A is provided by steam reforming and/or by autothermal reforming.

10. Process according to any of the preceding claims, wherein the synthesis of the urea in step (e) comprises the subreactions (i) and (ii):
(i) formation of ammonium carbamate from ammonia and carbon dioxide; and
(ii) conversion of the ammonium carbamate into urea and water;
where the energy for the endothermic subreaction (ii) is obtained at least partly from the exothermic subreaction (i).

11. Use of an apparatus for preparing urea, wherein the apparatus comprises the following interacting components:
(A) apparatus for providing a gas stream comprising hydrogen, nitrogen and carbon dioxide;
(B) means for removing (2) at least part of the carbon dioxide of the gas stream by means of a solvent;
(C) ammonia synthesis unit (5) comprising an ammonia reactor for the synthesis of ammonia from at least part of the hydrogen and at least part of the nitrogen which are present in the gas streams (1) and (4);
(D) means for desorbing the carbon dioxide (10) from the solvent; and
(E) urea synthesis unit (13) comprising a urea reactor for the synthesis of urea from at least part of the ammonia synthesized in the ammonia synthesis unit (5) and at least part of the carbon dioxide desorbed from the solvent;
where the means for desorbing the carbon dioxide (10) are operated at a higher pressure than the urea synthesis unit (13), in a process according to any of Claims 1 to 10.

12. Use according to Claim 11, wherein the means for desorbing the carbon dioxide (10) are operated at a pressure which is above the pressure at which the urea synthesis unit is operated and the pressure difference between the pressure at which the means for desorbing the carbon dioxide and the pressure at which the urea synthesis unit are operated is sufficiently high to compensate for any pressure drops of the carbon dioxide occurring before entry into the urea synthesis unit.

13. Use according to either of Claims 11 and 12, wherein the apparatus comprises a steam reformer and/or an autothermal reformer for providing a gas stream.

14. Use according to any of Claims 11 to 13, wherein the means for removing at least part of the carbon dioxide (2) comprise an ammonia-water scrub.

## Revendications

1. Procédé de fabrication d'urée, le procédé comprenant les étapes suivantes :
a) la mise à disposition d'un courant gazeux A (1) comprenant de l'hydrogène, de l'azote et du dioxyde de carbone ;
b) la séparation d'au moins une partie du dioxyde de carbone du courant gazeux A avec un solvant pour former un courant gazeux appauvri en dioxyde de carbone B (4) ;
c) la synthèse d'ammoniac à partir d'au moins une partie de l'hydrogène et d'au moins une partie de l'azote, qui sont contenus dans le courant gazeux B (4) ;
d) la désorption du dioxyde de carbone (12) à partir du solvant (3") ; et
e) la synthèse d'urée à partir d'au moins une partie de l'ammoniac synthétisé à l'étape (c) et d'au moins une partie du dioxyde de carbone désorbé à l'étape (d) ;
la désorption du dioxyde de carbone à l'étape (d) ayant lieu à une pression plus élevée que la synthèse de l'urée à l'étape (e).

2. Procédé selon la revendication 1, dans lequel le solvant chargé avec du dioxyde de carbone est au moins partiellement détendu après l'étape (b) et avant l'étape (d), afin d'éliminer les composants co-absorbés à l'étape (b) du solvant.

3. Procédé selon l'une quelconque des revendications précédentes, dans lequel la désorption du dioxyde de carbone à l'étape (d) a lieu à une pression plus élevée que la synthèse de l'urée à l'étape (e), et la différence de pression entre la pression à laquelle la désorption a lieu et la pression à laquelle l'urée est synthétisée à l'étape (e) est suffisamment élevée pour compenser des pertes de pression du dioxyde de carbone qui se produisent éventuellement jusqu'à l'entrée dans la synthèse de l'urée.

4. Procédé selon l'une quelconque des revendications précédentes, dans lequel la température à laquelle la désorption est réalisée à l'étape (d) est choisie de telle sorte que le dioxyde de carbone puisse être désorbé à la pression respective régnant.

5. Procédé selon l'une quelconque des revendications précédentes, dans lequel le solvant avec lequel le dioxyde de carbone est séparé à l'étape (b) comprend un mélange ammoniac/eau.

6. Procédé selon l'une quelconque des revendications précédentes, dans lequel la désorption du dioxyde de carbone à l'étape (d) a lieu à une température dans la plage allant de 130 °C à 270 °C.

7. Procédé selon l'une quelconque des revendications précédentes, dans lequel l'énergie nécessaire pour la désorption du dioxyde de carbone à l'étape (d) est mise à disposition par de la vapeur.

8. Procédé selon la revendication 7, dans lequel la vapeur qui doit être utilisée lors de la désorption du dioxyde de carbone est générée avec la chaleur de processus libérée lors de la synthèse d'ammoniac.

9. Procédé selon l'une quelconque des revendications précédentes, dans lequel au moins une partie du courant gazeux A est mise à disposition par reformage à la vapeur et/ou par reformage autothermique.

10. Procédé selon l'une quelconque des revendications précédentes, dans lequel la synthèse de l'urée à l'étape (e) comprend les réactions partielles (i) et (ii) :
(i) la formation de carbamate d'ammonium à partir d'ammoniac et de dioxyde de carbone ; et
(ii) la mise en réaction du carbamate d'ammonium pour former de l'urée et de l'eau ;
l'énergie pour la réaction partielle endothermique (ii) étant au moins partiellement couverte par la réaction partielle exothermique (i).

11. Utilisation d'un dispositif pour la fabrication d'urée, le dispositif comprenant les composants suivants en liaison active les uns avec les autres :
(A) un dispositif pour la mise à disposition d'un courant gazeux comprenant de l'hydrogène, de l'azote et du dioxyde de carbone ;
(B) des moyens pour la séparation (2) d'au moins une partie du dioxyde de carbone du courant gazeux avec un solvant ;
(C) une unité de synthèse d'ammoniac (5), comprenant un réacteur d'ammoniac pour la synthèse d'ammoniac à partir d'au moins une partie de l'hydrogène et d'au moins une partie de l'azote, qui sont contenus dans les courants gazeux (1) et (4) ;
(D) des moyens pour la désorption du dioxyde de carbone (10) à partir du solvant ; et
(E) une unité de synthèse d'urée (13), comprenant un réacteur d'urée pour la synthèse d'urée à partir d'au moins une partie de l'ammoniac synthétisé dans l'unité de synthèse d'ammoniac (5) et d'au moins une partie du dioxyde de carbone désorbé à partir du solvant ;
les moyens pour la désorption du dioxyde de carbone (10) étant exploités à une pression plus élevée que l'unité de synthèse d'urée (13), dans un procédé selon l'une quelconque des revendications 1 à 10.

12. Utilisation selon la revendication 11, dans laquelle les moyens pour la désorption du dioxyde de carbone (10) sont exploités à une pression qui est située au-dessus de la pression à laquelle l'unité de synthèse d'urée est exploitée, et la différence de pression entre la pression à laquelle les moyens pour la désorption du dioxyde de carbone sont exploités et la pression à laquelle l'unité de synthèse d'urée est exploitée est suffisamment élevée pour compenser les pertes de pression du dioxyde de carbone qui se produisent éventuellement jusqu'à l'entrée dans l'unité de synthèse d'urée.

13. Utilisation selon l'une quelconque des revendications 11 ou 12, dans laquelle le dispositif pour la mise à disposition d'un courant gazeux comprend un reformeur à la vapeur et/ou un reformeur autothermique.

14. Utilisation selon l'une quelconque des revendications 11 à 13, dans laquelle les moyens pour la séparation d'au moins une partie du dioxyde de carbone (2) comprennent un lavage ammoniac-eau.
